# EUROPEAN PATENT APPLICATION

(11) **EP 1 057 833 A1**
(43) Date of publication of application: **06.12.2000**
(21) Application number: 99903912.6
(22) Date of filing: 10.02.1999
(51) Int. Cl.: C08B 37/00

(54) **SULFATED SACCHARIDES**

(30) Priority: 17.02.1998 JP 4998998; 21.04.1998 JP 12541598
(71) Applicant: Takara Shuzo Co, Ltd., Kyoto-shi, Kyoto 612-8061 (JP)
(72) Inventor: SAKAI, Takeshi, Biomedical Group, Hirosaki-shi, Aomori 036-8216 (JP); KIMURA, Hitomi, Biomedical Group, Hirosaki-shi, Aomori 036-8216 (JP); KATAYAMA, Kaoru, Biomedical Group, Hirosaki-shi, Aomori 036-8216 (JP); SHIMANAKA, Kazuo, Central Research Laboratories, Otsu-shi, shiga 520-2193 (JP); IKAI, Katsushige, Central Research Laboratories, Otsu-shi, Shiga 520-2193 (JP); KATO, Ikunoshin, Central Research Laboratories, Otsu-shi, Shiga 520-2193 (JP)
(74) Representative: Gaunt, Robert John
(86) International application number: JP9900606
(87) International publication number: WO9941288

(57) **Abstract**

A sulfated saccharide represented by the following formula [I] or a sulfated polysaccharide where the said sulfated saccharide is an essential component of the constituting sugar as well as a salt thereof. 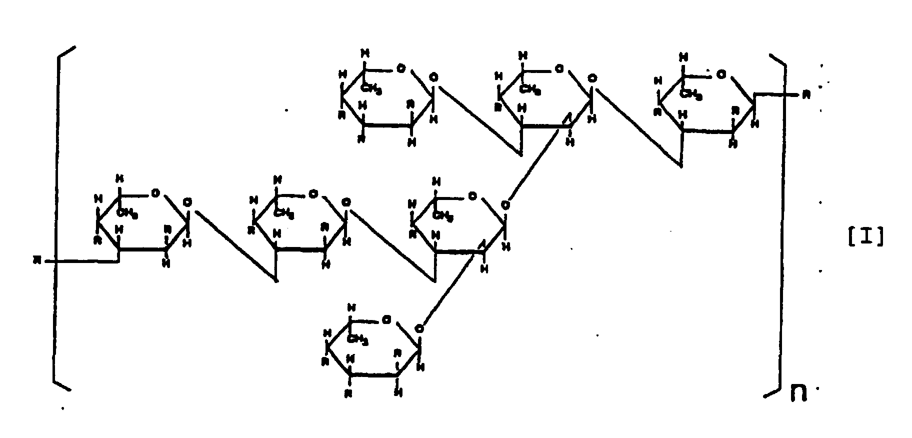 (In the formula, R is OH or OSO₃H; and n is an integer of 1-5.)

## Description

### TECHNICAL FIELD OF THE INVENTION

The present invention relates to sulfated saccharides and sulfated polysaccharides derived from marine algae which are useful as pharmaceuticals, reagents for the study of sugar chain engineering, cosmetics, etc.

### PRIOR ART

It has been known that marine algae belonging to brown algae contain sulfated polysaccharides and it has been reported that structure of fucoidan where sulfated-fucose is a main component of the constituting sugar considerably varies depending upon the type of the brown algae.

It has been also reported that the sulfated saccharides derived from sulfated polysaccharides considerably vary depending upon the type of the brown algae.

### PROBLEMS TO BE SOLVED BY THE INVENTION

A purpose of the present invention is to offer sulfated saccharides and sulfated polysaccharides having novel structures which are useful as reagents, pharmaceuticals, etc.

### MEANS TO SOLVE THE PROBLEMS

The present inventors have carried out an intensive investigation for sulfated polysaccharides derived from brown algae in order to achieve the above-mentioned purpose, have established a method for the manufacture of sulfated polysaccharides where a sulfated saccharide (hereinafter, just referred to as "sulfated polysaccharide of the present invention) represented by the following formula [I] as an essential component for the constituting sugar and the said sulfated saccharides and have accomplished the present invention.

In short, the present invention relates to a sulfated saccharide represented by the following formula [I] or to a sulfated polysaccharide where the said sulfated saccharide is an essential component of the constituting sugar as well as to a salt thereof. 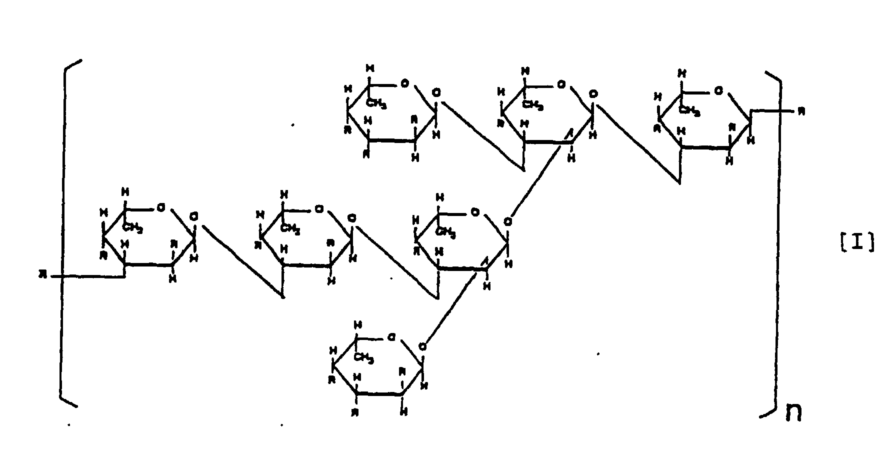 (In the formula, R is OH or OSO₃H; and n is an integer of 1-5,)

The second feature of the present invention relates to a method for the manufacture of a sulfated polysaccharide cross-linked with a polycationic substance which is characterized in including an extracting step under a non-destructive condition for a cross-link between polycationic substance and sulfated polysaccharide from algae.

The third feature of the present invention relates to a method for the manufacture of a sulfated polysaccharide cross-linked with a polycationic substance which is characterized in adding a polycationic substance having higher isoelectric point than the pH of a solution of sulfated polysaccharide to the said solution.

The fourth feature of the present invention relates to a method of reinforcing a viscoelasticity of a sulfated polysaccharide, characterized in that, a polycationic substance is added to a sulfated polysaccharide.

The fifth feature of the present invention relates to a composition which contains at least a sulfated polysaccharide having a low viscoelasticity and a polycationic substance.

The sixth feature of the present invention relates to a pharmaceutical agent, characterized in that, a sulfated polysaccharide which is cross-linked with a polycationic substance is contained as an effective component.

The seventh feature of the present invention relates to a lubricant, characterized in that, a sulfated polysaccharide which is cross-linked with a polycationic substance is contained as an effective component.

The eighth feature of the present invention relates to cosmetics, characterized in that, a sulfated polysaccharide which is cross-linked with a polycationic substance is contained as an effective component.

### BRIEF EXPLANATION OF THE DRAWINGS

Fig. 1 shows the result of mass spectrometry of the sulfated saccharide.
Fig. 2 shows a ¹H-NMR spectrum of the sulfated saccharide.
Fig. 3 shows a ¹³C-NMR spectrum of the sulfated saccharide.
Fig. 4 shows the ¹H-NMR spectrum of the fraction no. 67.
Fig. 5 shows a ¹H-NMR spectrum of the sulfated polysaccharide according to the present invention.
Fig. 6 shows an IR spectrum of the sulfated polysaccharide according to the present invention.

### BEST MODE FOR CARRYING OUT THE INVENTION

The present invention will now be specifically illustrated as hereunder.

With regard to the type of the brown algae used in the present invention, there is no particular limitation so far as it contains the sulfated polysaccharide of the present invention. Marine algae belonging to Order of *Laminariales* such as *Kjellmaniella crassifolia*, *Laminaria japonica, Undaria* *pinnatifida*, *Ecklonia kurome, Eisenia bicyclis, Ecklonia cava,* giant kelp, *Lessonia nigrescens*, etc. are the particularly preferred starting materials since they contain a lot of the sulfated polysaccharide of the present invention.

In the manufacture of the sulfated polysaccharide of the present invention, an extract of the sulfated polysaccharide is obtained from brown algae using an aqueous solvent. The marine algae used for the extraction may be raw algae or the brown algae may be dried or made into dried powder before obtaining the extract.

Moreover, when the dried algae material is washed with 60-100% alcohol, acetone or the like, contamination of coloring substances into the sulfated polysaccharide of the present invention is significantly reduced and, therefore, that is advantageous in the manufacture of the sulfated saccharide of the present invention which will be carried out later.

Extraction of the sulfated polysaccharide from brown algae according to the present invention is preferably carried out in the presence of ethyl alcohol and the sulfated polysaccharide of the present invention can be selectively extracted with an aqueous solvent preferably in the presence of 5-40% ethyl alcohol or, more preferably, 8-15% ethyl alcohol.

Further, when an inorganic salt which forms a precipitate with alginic acid such as calcium chloride or calcium acetate is used in the extraction of the sulfated polysaccharide of the present invention from brown algae, contamination of alginic acid is greatly reduced and, therefore, that is advantageous for the purification which will be conducted thereafter.

Temperature for extracting the sulfated polysaccharide of the present invention is preferably 50°C or lower or, advantageously, 15-30°C.

The pH upon extraction may depend upon the temperature but, since the sulfated polysaccharide of the present invention is unstable to acid and alkali, an extraction at the neutral region of about pH 6-8 is preferred.

The extraction may be carried out with stirring but, most advantageously, it is carried out under a non-shearing condition whereby the sulfated polysaccharide of the present invention can be efficiently prepared.

Incidentally, the above-mentioned extract of brown algae is often contaminated with impurities such as neutral sugars and proteins. Removal of neutral sugars can be easily achieved by means of an ultrafiltration where the excluding molecular weight is about 100,000 or less. In removing the proteins, a treatment with protease, etc. is effective.

In the manufacture of the sulfated saccharide of the present invention which is a low-molecular substance from the sulfated polysaccharide of the present invention, the said sulfated polysaccharide is treated with an endo-sulfated polysaccharide degrading enzyme which has an action of liberating the sulfated saccharide of the present invention by a selective action to the said polysaccharide. There is no particular limitation for the said endo-sulfated polysaccharide degrading enzyme and its example is an endo-sulfated polysaccharide degrading enzyme produced by *Alteromonas* sp. SN-1009 (FERM BP-5747) which will be mentioned in Example 1-(1) later.

It is possible that the sulfated polysaccharide of the present invention is treated with the above endo-sulfated polysaccharide degrading enzyme and then the resulting reaction product is used as it is but, when the product is used as a pharmaceutical agent or a reagent, it is recommended to purify the sulfated saccharide of the present invention from the reaction solution. Ultrafiltration may be applied or anionic exchange resin, resin for hydrophobic chromatography, resin for gel filtration, etc. may be used in conducting the purification.

The sulfated saccharide of the present invention may be purified from a degraded product which is obtained by a direct treatment of the raw brown algae with the above-mentioned endo-sulfated polysaccharide degrading enzyme or may be purified from a degraded product which is obtained by a treatment of dried marine algae, alcohol-washed marine algae, a substance containing the sulfated polysaccharide of the present invention, etc. with the above-mentioned endo-sulfated polysaccharide degrading enzyme. Incidentally, the efficiency of production of the sulfated saccharide of the present invention is improved when a step where the sulfated saccharide of the present invention is separated from the reaction solution is combined in the above enzymatic reaction.

Examples of the sulfated saccharide of the present invention are various kinds of sulfated saccharides where n is 1-5 in the formula [I] and the sulfated saccharides having various molecular weights can be prepared depending upon the condition for the enzymatic action where the sulfated polysaccharide of the present invention is treated with the above-mentioned endo-sulfated polysaccharide degrading enzyme. Each of the resulting sulfated saccharides may be isolated by the above purifying means from fractionated product obtained by a molecular weight fractionation of the sulfated saccharides by, for example, means of gel filtration or ultrafiltration. An example of the gel filtration is that Cellulofine GCL-300 is used for preparing the fractions with molecular weight of, for example, more than 25,000, more than 10,000 up to 25,000, more than 5,000 up to 10,000, etc. and another example is that Cellulofine GCL-90 is used for separating a fraction with molecular weight of 20,000 or less into fractions with molecular weights of, for example, more than 15,000 up to 20,000, more than 10,000 up to 15,000, more than 5,000 up to 10,000, etc.

In the case of ultrafiltration, a molecular weight fractionation may be carried out using, for example, ultrafiltration membrane and holofiber for ultrafiltration. For instance, a fraction of molecular weight of 30,000 or less and that of 6,000 or less can be prepared using FE10-FUS0382 and FE-FUS-653 (both manufactured by Daicel), respectively. When such gel filtration and ultrafiltration are combined, fraction of any molecular weights can be prepared and the desired sulfated saccharide can be isolated from the fraction prepared as such. Accordingly, the sulfated saccharide of the present invention covers the sulfated saccharide where n is more than 5 in the formula [I] as well.

With regard to salts of the sulfated polysaccharide and of the sulfated saccharide of the present invention, there are pharmaceutically acceptable salts and they can be prepared by a known method.

The sulfated saccharide, the sulfated polysaccharide or salt thereof obtained by the present invention has very high fucose residue content and sulfated degree thereof and is useful as a reagent for studying structures of sulfated-fucose-containing polysaccharides and also as a reagent for studying a physiological function of sulfated-fucose-containing polysaccharide.

With regard to the sulfate group in the sulfated saccharide of the present invention and the sulfated polysaccharide of the present invention, numbers of the sulfate group may vary depending upon the extracting condition for the sulfated polysaccharide and the manufacturing condition for the sulfated saccharide. For example, ester bond of the sulfate group is usually weak to acid while the sulfate esters bonded to 2- and 3-positions of fucose are weak to alkali. Thus, there is no particular limitation for the numbers of the sulfate group in the sulfated saccharide of the present invention and the sulfated polysaccharide of the present invention but, by setting due conditions, any sulfate group content is available.

The sulfated saccharide, the sulfated polysaccharide or a salt thereof according to the present invention alto exhibits a hydrophobicity due to a methyl group in the fucose residue and, therefore, it has a high affinity to various basic organic substances and protein. Therefore, the sulfated saccharide, the sulfated polysaccharide or the salt thereof according to the present invention has not only a high water-holding ability but also a high affinity to, for example, keratinous layer due to its hydrophobicity and charge whereby the sulfated saccharide, the sulfated polysaccharide or the salt thereof according to the present invention is quite useful as a material for cosmetics.

The sulfated saccharide, the sulfated polysaccharide or the salt thereof according to the present invention can be used by mixing with any substance which is applicable as cosmetics. Generally, it can be used as lotion, milky lotion, cream, etc. by mixing with water, alcohol, fat/oil, fatty acid, glycerol, inorganic salt, antiseptic agent, surface-active agent, vitamins, amino acids, saccharides, etc.

The substance containing the sulfated polysaccharide of the present invention is extracted from crude or dried marine algae belonging to brown algae such as *Kjellmaniella crassifolia, Laminaria japonica, Undaria pinnatifida, Ecklonia kurome,* giant kelp, *Lessonia nigrescens,* etc. or a product obtained by washing the above with a solution containing 60% or more organic solvent such as ethanol (all of them will be referred to as "marine algae material" hereinafter). Water, an aqueous solution of salt such as potassium chloride and sodium chloride or/and organic solvent such as 40% or less ethanol, etc. may be used as an extracting liquid. For example, the above marine algae material is dipped in the above extracting liquid and allowed to stand at 50°C or lower or stirred to manufacture the substance containing the sulfated polysaccharide of the present invention. There is no particular limitation for the substance containing the sulfated polysaccharide of the present invention so far as it contains the sulfated polysaccharide where the sulfated saccharide of the present invention is an essential component of the constituting sugar and, in the resulting substance containing the sulfated polysaccharide of the present invention, polysaccharides, alginic acid, amino acids, sugar alcohols, fat/oil, inorganic salts, proteins, etc. may be further contained in addition to the sulfated polysaccharide of the present invention. The substance containing the sulfated polysaccharide of the present invention shows virus infection-inhibiting action, fertilization-inhibiting action, anti-inflammatory action, suppressing action to thickening of hemangioendothelium, suppressing action to formation of thrombus, clearing action to lipemia, lowering action to serum cholesterol, anti-allergic action, etc. due to the physiological activity of the sulfated polysaccharide of the present invention and is useful as a material for pharmaceuticals. Due to the physiological activity of the sulfated polysaccharide of the present invention, the substance containing the sulfated polysaccharide of the present invention shows moisturizing action, inhibiting action to melanin dye synthesis, etc. and is useful as a material for cosmetics. An example of the sulfated polysaccharide of the present invention contained in the substance containing the sulfated polysaccharide of the present invention is a sulfated polysaccharide containing fucose as constituting sugar and about two molecules of sulfate group per molecule of fucose. The sulfate group of the said sulfated polysaccharide can be bonded with cation existing in sea water or an extract such as sodium, potassium, calcium, magnesium, zinc, manganese, iron, etc. as a counter ion. The said sulfated polysaccharide extracted from marine algae is usually cross-linked with a protein derived from marine algae showing a strong viscoelasticity and, therefore, measurement of molecular weight by means of gel filtration is not appropriate. However, when it is stirred for one hour or longer under the high concentration of a salt such as 1M or higher sodium chloride, the cross-link can be cleaved and it is possible to measure the molecular weight of the said sulfated polysaccharide. When pullulan is used as a standard substance, the molecular weight is measured to be about 10,000,000.

The substance containing the sulfated polysaccharide of the present invention also covers a sulfated polysaccharide cross-linked with a polycationic substance which is manufactured as follows. Thus, a sulfated polysaccharide cross-linked with a polycationic substance which is manufactured by a method including a step where an extraction from algae is conducted under a non-destructive condition for the cross-link of the polycationic substance with the sulfated polysaccharide or a solution of the said sulfated polysaccharide and then a polycationic substance having a higher isoelectric point than the pH of the said solution is added thereto.

Examples of the property of the sulfated polysaccharide are water-absorbing property, water-holding property, viscosity, thread forming property and viscoelasticity and they greatly vary depending upon a little difference in the extracting condition from the starting material for example. Although those properties are very useful for use as cosmetics, lubricant and moisturizer, it has been difficult to manufacture the sulfated polysaccharide with a good reproducibility and to control those properties.

Thus, when the sulfated polysaccharide is utilized as cosmetics, lubricants, moisturizers or certain types of pharmaceuticals, good feel on use and reproducibility of the property are not available causing a big problem upon putting in the market unless reproducibility in water-absorbing property, water-holding property, viscoelasticity, etc. are good. In accordance with the present invention however, it is now possible to offer a substance containing the sulfated polysaccharide of the present invention where water-absorbing property, water-holding property, viscoelasticity, etc. are stable, a method for manufacturing the same and the use thereof.

Thus, the present inventors have carried out an intensive study for an interaction between the sulfated polysaccharide of the present invention and a polycation such as protein and have found that there is a significant increase in viscoelasticity when a sulfated polysaccharide coexists with a polycationic substance under a certain condition.

The present inventors have moreover found that the sulfated polysaccharide contained in brown algae in its natural forms cross-links with the protein contained in the brown algae and is available as a substance containing a sulfated polysaccharide having a high viscoelasticity together with maintaining its cross-link under a certain condition.

There is no particular limitation for the sulfated polysaccharide in the present invention and its examples are sulfated-fucose-containing polysaccharide, dextran sulfate, carrageenan, heparin, rhamnan sulfate and chondroitin sulfate.

There is no particular limitation for the polycationic substance in the present invention and it covers polycationic substances such as polypeptide, protein, polyamine, polyethyleneimine, polyglucosamine and polygalactosamine.

Viscoelasticity of the sulfated polysaccharide is significantly high when a cross-linking of a sulfated polysaccharide with a polycationic substance is carried out under such conditions that the pH is higher than the isoelectric point of the sulfated polysaccharide used, that the pH is lower than the isoelectric point of the polycationic substance used, that the concentration of the coexisting electrolyte is low enough whereby the cross-linking of the sulfated polysaccharide with the polycationic substance used is not disturbed and that the amount of the polycationic substance added is less than the amount for neutralizing the total charge of the sulfated polysaccharide.

When protein having a relatively high isoelectric point such as gelatin or collagen is used as a polycationic substance, there are many positive charges and they are apt to cross-link the sulfated polysaccharide and, therefore, viscoelasticity is easily given. However, even in the case of protein having a relatively low isoelectric point, it is possible to give a strong viscoelasticity to the sulfated polysaccharide like in the case of gelatin and collagen if the pH of the sulfated polysaccharide solution is made lower than the isoelectric point of the protein used.

The sulfated polysaccharide which is cross-linked with a polycationic substance according to the present invention can be prepared from algae. For example, in the case of sulfated-fucose-containing sulfated polysaccharide contained in brown algae, its extracting efficiency and property considerably vary depending upon the method for extraction.

If a purpose is just to obtain a large amount of sulfated-fucose-containing sulfated polysaccharide within a short period, the sulfated-fucose-containing sulfated polysaccharide can be easily extracted when the algae are pulverized and extracted with water or with an aqueous solution of acid, salt, alkali or the like with heating and stirring.

However, the sulfated-fucose-containing sulfated polysaccharide prepared as such has less molecular weight and shows nearly no viscoelasticity as compared with that in a form existing in algae. Accordingly, when it is used for cosmetics, although the effect as a sulfated saccharide is available, there is no characteristic feature such as sliminess and lubricity and it is nearly impossible to use it as a lubricant as well.

However, when algae are dipped in water or an aqueous solution near a neutral state at the temperature of not higher than 50°C or, preferably, not higher than about 30°C and the stirring speed is made to such an extent that a shearing force is hardly resulted or, for example, that the extracted substance having a strong viscoelasticity is not detached from the algae, it is now possible that a sulfated-fucose-containing sulfated polysaccharide is extracted in a form of being cross-linked with the protein in the algae.

The sulfated-fucose-containing sulfated polysaccharide which is cross-linked with protein as such has a property like fresh egg white, i.e. a viscoelasticity.

The cross-link between protein and sulfated-fucose-containing sulfated polysaccharide is dissociated relatively easily. When the sulfated-fucose-containing sulfated polysaccharide which cross-linked with protein is stirred, the liquid is wound up along the stirring axis (a Weissenberg effect) but, when a stirring where a shearing force is strongly applied is carried out for a long period, the viscoelasticity lowers and the liquid surface becomes rather hollow at the area contacting with the stirring axis. Moreover, the cross-link between protein and sulfated-fucose-containing sulfated polysaccharide is dissociated upon heating, addition of salt of high concentration, treatment with protease, etc. whereupon the viscoelasticity disappears.

Thus, the strong viscoelasticity of the sulfated-fucose-containing sulfated polysaccharide cross-linked with protein which is extracted as above is not inherent to the sulfated-fucose-containing sulfated polysaccharide but is a property which is available when the sulfated-fucose-containing sulfated polysaccharide and protein are cross-linked.

Accordingly, it is possible to recover the viscoelasticity for the sulfated-fucose-containing sulfated polysaccharide which once lost its viscoelasticity and, when a polycationic substance such as protein is added to the said sulfated-fucose-containing sulfated polysaccharide, the viscoelasticity can be easily recovered. It is also possible to adjust the degree of the viscoelasticity to any extent by changing the adding amount of the polycationic substance such as protein. Thus, the present invention offers a method of potentiating the viscoelasticity of the sulfated polysaccharide which is characterized in adding a polycationic substance to the sulfated polysaccharide.

For example, when a mixing is carried out under the condition which is in a little lower pH than the isoelectric point of a polycationic substance such as protein in such an amount that the charge of the sulfated polysaccharide such as sulfated-fucose-containing sulfated polysaccharide is not neutralized, there is a positive correlation between the mixing amount of the polycationic substance such as protein and the viscoelasticity of a solution of the sulfated-fucose-containing sulfated polysaccharide.

It goes without saying that a precipitate is produced and the viscoelasticity lowers when pH of the solution of the sulfated polysaccharide such as sulfated-fucose-containing sulfated polysaccharide is far lower than the isoelectric point of the polycationic substance to be added or when the amount of the polycationic substance to be added is so high that it neutralizes the charge of the sulfated-fucose-containing sulfated polysaccharide.

There is no particular limitation for the marine algae used for obtaining the sulfated-fucose-containing sulfated polysaccharide cross-linked with protein and any type of brown algae may be used so far as it contains the sulfated-fucose-containing sulfated polysaccharide such as *Kjellmaniella crassifolia, Kjellmaniella gyrata* (tangle flakes), *Laminaria japonica, Ecklonia cava, Eisenia bicyclis, Undaria pinnatifida, Nemacystus decipiens, Cladosiphon okamuranus,* etc.

The brown algae may be used in any of the forms such as raw marine algae, dried marine algae, salted marine algae, etc.

When the marine algae are washed with an alcohol of about 60-100% before extraction of the sulfated-fucose-containing sulfated polysaccharide cross-linked with protein according to the present invention, salts, coloring substances, etc. adhered to the marine algae can be removed. In the extraction of the sulfated-fucose-containing sulfated polysaccharide cross-linked with protein according to the present invention, various solvents such as water, salt-containing water, alcohol-containing water, etc. may be used. However, salt has an action of dissociating the cross-link between the sulfated-fucose-containing sulfated polysaccharide with protein resulting in a decrease in its viscoelasticity and, therefore, the salt is preferably 100 mM or less. With regard to alcohol, it is preferred to be 30% or less.

The extracting temperature is preferably 50°C or lower or, advantageously, 5-30°C. Stirring during the extraction may not be carried out but, when the extracting solution is still, it is necessary to conduct the extraction for a long period. When stirring is carried out, the stirring speed is selected to such an extent that the viscoelasticity of the sulfated-fucose-containing sulfated polysaccharide cross-linked with protein is not too much lowered as mentioned already. However, the strength of the shearing force has far more influence on the viscoelasticity than the stirring speed and, therefore, it is preferred that the extraction is carried out by a low stirring speed under a non-shearing condition.

Incidentally, recovery of the reduced viscoelasticity by stirring can be done by adding a polycationic substance such as gelatin or collagen.

Thus, when a polycationic substance is added to the sulfated polysaccharide having a low viscoelasticity, the viscoelasticity of the sulfated polysaccharide can be increased and a composition which contains at least the sulfated polysaccharide having a low viscoelasticity and a polycationic substance as constituting elements can be offered. The said composition is useful in various uses including cosmetics and lubricants as a composition having a strong viscoelasticity.

With regard to the sulfated polysaccharide cross-linked with a polycationic substance, an extract may be used as it is or after further purification.

A pharmaceutical agent containing the sulfated polysaccharide cross-linked with a polycationic substance according to the present invention as an effective component such as an intraoral preparation can be manufactured where the sulfated polysaccharide cross-linked with a polycationic substance according to the present invention is used as an effective component and is combined with a known pharmaceutical carrier.

When the intraoral preparation of the present invention is kept in one's mouth in case secretion of saliva is so insufficient that mouth becomes dry and opening-and-shutting of the mouth is difficult, the symptom can be significantly improved.

Moreover, the sulfated polysaccharide cross-linked with a polycationic substance according to the present invention can be used as a lubricant. Such a lubricant of the present invention is very smooth, has a good lubricating action and shows a very good lubricating action when various medical instruments or pharmaceuticals are inserted into anus or vagina. It can be used as a lubricant during sexual intercourse or massage as well.

The sulfated polysaccharide cross-linked with a polycationic substance according to the present invention can be used by mixing with any substance which can be used as cosmetics. Generally, it is mixed with water, alcohol, fat/oil, fatty acid, glycerol, inorganic salt, antiseptic agent, surface-active agent, vitamin, amino acid, saccharide, etc. and can be used as lotion, milky lotion, cream, etc.

When the sulfated polysaccharide cross-linked with a polycationic substance according to the present invention is used as cosmetics, it shows a smooth and good feel on use due to its viscoelasticity and, when applied to skin, its sliminess is immediately adsorbed to the skin. Thus, there is no stickiness after application and that is a very desirable property as cosmetics. When a sulfated-fucose-containing sulfated polysaccharide is used as a sulfated polysaccharide, the above-mentioned preferred property is particularly remarkable.

The sulfated saccharide of the present invention and the sulfated polysaccharide of the present invention can be used as antigens. Preparation of the antibody can be carried out by a common method and, for example, the sulfated saccharide of the present invention or the sulfated polysaccharide of the present invention is immunized to animals such as rabbit together with an adjuvant whereupon polyclonal antibody can be prepared. With regard to monoclonal antibody, it can be prepared by such a manner that melanoma cells are fused with antibody-producing B cells obtained by immunization of an antigen to select a hybridoma which produces the desired antibody and then the said cells are incubated. The antibodies prepared as such can be used for purification of the sulfated saccharide of the present invention or the sulfated polysaccharide of the present invention. Moreover, they can be used for identification of the sulfated polysaccharide of the present invention in marine algae. For example, when the antibody of the present invention is used, content of the sulfated polysaccharide of the present invention in marine algae extract can be easily measured whereby it is possible to efficiently prepare the extract containing a high content. Thus, it is found that the sulfated polysaccharide of the present invention is highly contained in the extract of Kjellmaniella *crassifolia, Lessonia nigrescens, Laminaria japonica,* etc. for example whereby the industrial production of the sulfated polysaccharide can be efficiently carried out. Moreover, the antibody which recognizes the sulfated saccharide of the present invention or the sulfated polysaccharide of the present invention is useful for clarifying the physiological function of the sulfated saccharide of the present invention or the sulfated polysaccharide of the present invention. Thus, for example, it is useful for the analysis of function of fertilization inhibiting action, function of viral infection inhibiting action, metabolism in *vivo*, etc. of the sulfated saccharide of the present invention or the sulfated polysaccharide of the present invention.

The sulfated saccharide of the present invention is useful as a substance for sugar chain engineering and, when it is subjected to a 2-aminopyridylation by a method disclosed in the Japanese Laid-Open Patent Publication Hei-05/65108 to prepare a 2-aminopyridyl derivative thereof, it is now possible to offer a substance which is quite useful as a substance for sugar chain engineering.

Moreover, it is possible to manufacture pharmaceuticals containing the sulfated saccharide of the present invention and/or the sulfated polysaccharide of the present invention as effective component(s) such as anti-tumor agent, an inhibitor of cancer metastasis, antiviral agent, fertilization inhibitor, anti-inflammatory agent, inhibitor of intimal hyperplasia, inhibitor for thrombus formation, clearing agent for lipemia, serum cholesterol lowering agent, etc. They can be used for therapy or prevention of those diseases which need administration of those pharmaceuticals. The substance containing the sulfated polysaccharide of the present invention can be used as a material for those pharmaceuticals as well. It is also possible to offer food or beverage having the above-mentioned physiological activity where a substance selected from the sulfated saccharide of the present invention, the sulfated polysaccharide of the present invention and the substance containing the sulfated polysaccharide of the present invention is contained therein, diluted therewith and/or added thereto. It is moreover possible to offer cosmetics containing a substance selected from the sulfated saccharide of the present invention, the sulfated polysaccharide of the present invention and the substance containing the sulfated polysaccharide of the present invention as an effective component.

### EXAMPLES

The present invention will now be more specifically illustrated by way of the following examples although the present invention is not limited to the coverage of those examples only.

### Example 1.

(1) *Alteromonas* sp. SN-1009 (FERM BP-5747) was inoculated to a two-liter Erlenmeyer's flask containing 600 ml of a medium (sterilized at 120°C for 20 minutes) (pH 8.2) consisting of artificial sea water (manufactured by Jamarin Laboratory) containing 0.25% of glucose, 1.0% of peptone and 0.05% of yeast extract and incubated at 25°C for 26 hours to give a seed culture medium. A medium (20 liters) consisting of artificial sea water (pH 8.0) containing 1.0% of peptone, 0.02% of yeast extract, 0.2% of the sulfated polysaccharide mentioned in the following Example 2 and 0.01% of antifoaming agent (KM 70 manufactured by Shin-Etsu Chemical Industry) was placed in a 30-liter jar fermenter and sterilized at 120°C for 20 minutes. After cooling, 600 ml of the above-prepared seed culture medium were inoculated and incubated at 24°C for 24 hours under the condition of 10 liters of aeration per minute and stirring of 250 rpm. After completion of the incubation, the medium was centrifuged to give cells and supernatant liquid. The resulting supernatant liquid was concentrated by an ultrafiltration system equipped with a holofiber having an excluding molecular weight of 10,000 and salted out by ammonium sulfate (85% saturation) and the resulting precipitate was collected by centrifugation and fully dialyzed against 20mM of Tris-HCl buffer (pH 8.2) containing 1/10 concentration of artificial sea water to give 600 ml of an endo-sulfated polysaccharide degrading enzyme selectively acting on the sulfated polysaccharide of the present invention.

(2) Dried *Kjellmaniella crassifolia* (2 kg) was powdered by a cutter mill (manufactured by Masuko Sangyo) equipped with a screen having a mesh of 1mm diameter, the resulting chips of the sea tangle were suspended in 20 liters of 80% ethanol, stirred at 25°C for three hours, filtered using a filter paper and the residue was well washed. The resulting residue was suspended in 20 liters of buffer (pH 8.2) containing the endo-sulfated polysaccharide degrading enzyme prepared in the above Example 1-(1), 10% of ethanol, 100 mM sodium chloride, 50 mM of calcium chloride and 50 mM of imidazole and the suspension was stirred at 25°C for 48 hours. The suspension was filtered through a stainless wire net having a mesh of 32 µm and the residue was washed with 10% ethanol containing 50 mM of calcium chloride. The residue was further suspended in 10 liters of 10% ethanol containing 50 mM calcium chloride, stirred for three hours and filtered through a stainless wire net followed by washing. After that, the residue was suspended under the same condition again, stirred for 16 hours and filtered through a stainless wire net of 32 µm diameter followed by washing.

The resulting filtrate and washing were collected and subjected to an ultrafiltration using an ultrafiltration system equipped with a holofiber having an excluding molecular weight of 3,000 to separate into a filtrate and a non-filtrate.

The filtrate was concentrated to about three liters using a rotary evaporator and centrifuged to give a supernatant liquid. The resulting supernatant liquid was desalted using an electric dialyzer equipped with a membrane having an excluding molecular weight of 300, calcium acetate was added thereto to make its concentration 0.1M and the resulting precipitate was removed by centrifugation. The supernatant liquid was put on an DEAE-Cellulofine (resin amount: 4 liters) previously equilibrated with 50 mM of calcium acetate, well washed with 50 mM of calcium acetate and 50 mM of sodium chloride and eluted with sodium chloride with a gradient of from 50 mM to 800 mM. The collecting amount at that time was 500 ml per elution. The collected fraction was analyzed by a cellulose acetate membrane electrophoretic method [*Analytical Biochemistry*, **37**, 197-202 (1970)] whereupon it was found that the eluted sulfated saccharide where the sodium chloride concentration was about 0.4M (near the fraction no. 63) was homogeneous. Moreover, the sulfated saccharide eluted at the concentration of about 0.6M (near the fraction no. 67) was electrophoretically almost homogeneous.

Therefore, firstly, the liquid of the fraction no. 63 was concentrated to 150 ml, sodium chloride was added to make its concentration 4M, put on Phenyl-Cellulofine (resin amount: 200 ml) previously equilibrated with 4M sodium chloride and well washed with 4M sodium chloride. The non-adsorptive sulfated saccharide fractions were collected and desalted by an electric dialyzer equipped with a membrane having an excluding molecular weight of 300 to give 505 ml of a desalted solution.

Forty ml of the resulting desalted solution were put on a column (4.1 cm × 87 cm) of Cellulofine GCL-90 equilibrated with 0.2M sodium chloride containing 10% ethanol to conduct a gel filtration. Collection was carried out at the rate of 9.2 ml per fraction.

Analysis of the total saccharide amount in the total fractions was carried out by a phenol-sulfuric acid method (*Analytical Chemistry*, **28**, 350 (1956)].

As a result thereof, the sulfated saccharide formed a peak and, therefore, the central part of the said peak (fraction nos. 63-70) was collected, desalted by an electric dialyzer equipped with a membrane having an excluding molecular weight of 300 and freeze-dried to give 112 mg of a dried product of the sulfated saccharide of the present invention.

A part of the dried product was subjected to a sugar composition analysis and a mass spectrometry.

Moreover, 10mg of the dried product were substituted with heavy water by a common method and subjected to an NMR analysis.

As a result of the sugar composition analysis, the resulting sulfated saccharide was found to be a sulfated saccharide consisting of fucose only.

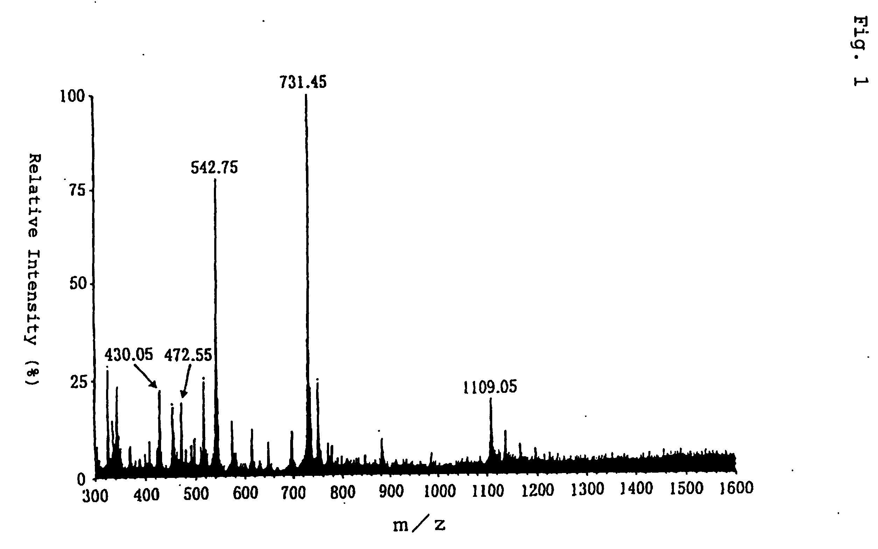

Result of the mass spectrometry for the sulfated saccharide using an API-III Mass Spectrometer (Perkin-Elmer Sciex) is shown in Fig. 1 while the analytical result is given as hereunder. Thus, Fig. 1 is a drawing which shows the result of mass spectrometry of the sulfated saccharide where the ordinate indicates a relative intensity (%) while the abscissa indicates m/z values.

With regard to a molecular weight, a result of 2264 ± 1 was obtained in a state where all sulfate groups are in sodium salt. Thus, since this is a sulfated saccharide where the constituting sugar is fucose only, it has been found that 7 molecules of fucose and 12 molecules of sulfate group are bonded, that all of the sulfate groups are in sodium salt and that the theoretical molecular weight is 2265.

Thus, when the present substance is expressed by "M", main signals in Fig. 1 can be assigned to be as follows.

| | |
|---|---|
| m/z | 1109.05 --- [M-2Na⁺]²⁻ (calculated: 1109.5) |
| | 731.45 --- [M-3Na⁺]³⁻ (calculated: 732) |
| | 542.75 --- [M-4Na⁺]⁴⁻ (calculated: 543.25) |
| | 430.05 --- [M-5Na⁺]⁵⁻ (calculated: 430) |

As a result thereof, the present substance is an oligosaccharide consisting of 7 molecules of fucose and 12 molecules of sulfate group.

Then, in order to determine the bonding forms of fucose and the binding positions of the sulfate groups, an NMR analyses were carried out using a nucleomagnetic resonance meter (JNM-α 500; manufactured by Nippon Denshi). Bonding forms of the constituting sugars were analyzed by an HMBC method which is a ¹H-detecting heteronucleus detection method. For assignment of ¹H-NMR, a DQF-COSY method and an HOHAHA method were used and, for assignment of ¹³C-NMR, an HSQC method was used.

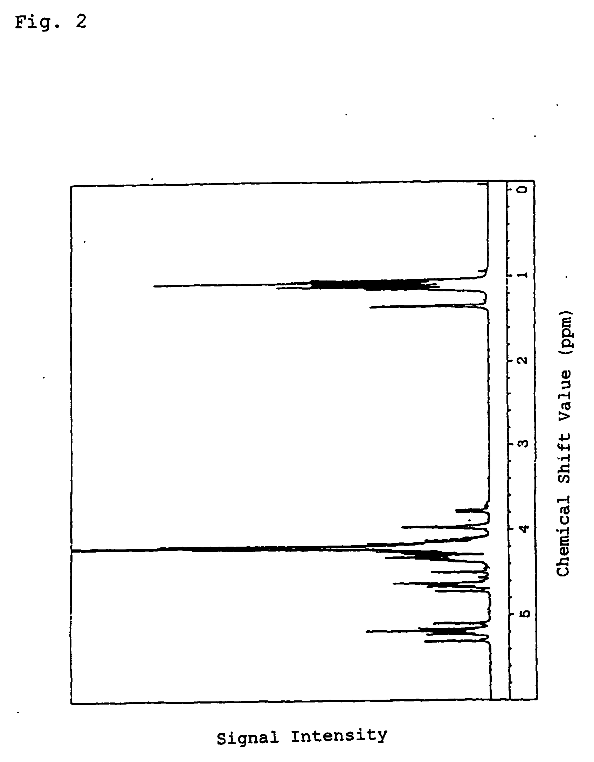

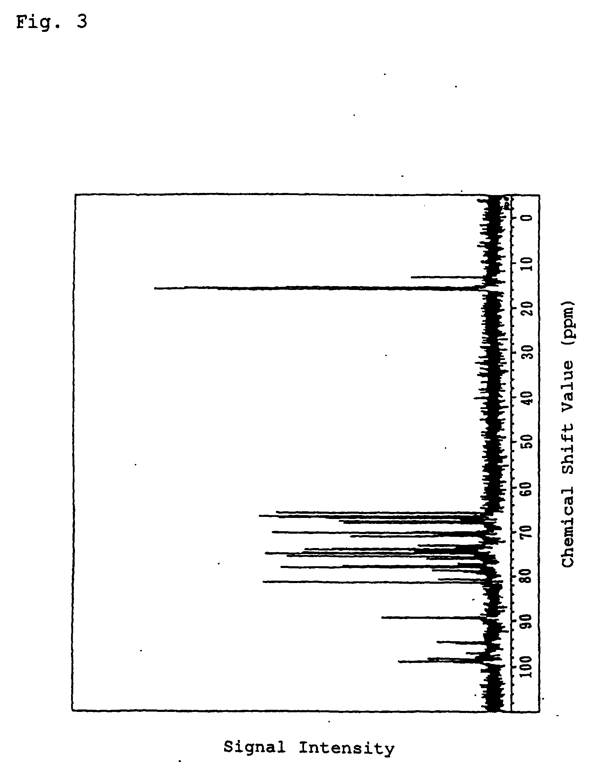

Result of assignment of NMR is shown as hereunder and ¹H-NMR spectrum and ¹³C-NMR spectrum of the sulfated saccharide of the present invention are shown in Fig. 2 and Fig. 3, respectively. Incidentally, with regard to the chemical shift value in ¹H-NMR, the chemical shift value of dioxane was expressed as 3.53 ppm while, in the case of ¹³C-NMR, the chemical shift value of dioxane was expressed as 66.5 ppm. Measurement was carried out at 60°C in both cases. Thus, Fig. 2 is a graph showing the ¹H-NMR spectrum of the sulfated saccharide of the present invention while Fig. 3 is a graph showing the ¹³C-NMR spectrum of the sulfated saccharide of the present invention. In both Fig. 2 and Fig. 3, the ordinate and the abscissa indicate a signal intensity and a chemical shift value (ppm), respectively.

### ¹H-NMR(D₂ O)

δ 5.30 (1H, d, J=3.1Hz, A-1-H), 5.23 (1H, d, J=3.4Hz, B-1-H), 5.20 (1H, d, J=3.4Hz, E-1-H), 5.19 (1H, d, J=3.7Hz, F-1-H), 5.18 (1H, d, J=2.8Hz, C-1-H), 5.16 (1H, br-s, D-1-H), 5.09 (1H, d, J=4.3Hz, G-1-H), 4.72 (1H, d, J=2.4Hz, B-4-H), 4.67 (1H, t, J=2.3Hz, A-4-H), 4.65 (1H, m, E-4-H), 4.64 (1H, m, D-4-H), 4.62 (1H, m, C-4-H), 4.49 (1H, d, J=3.1Hz, F-4-H), 4.37 (1H, m, E-2-H), 4.36 (1H, m, G-3-H), 4.35 (1H, m, C-2-H), 4.33 (1H, m, B-2-H), 4.32 (1H, m, C-3-H), 4.30 (1H, m, A-2-H), 4.27 (1H, m, F-2-H), 4.27 (1H, m, F-5-H), 4.25 (1H, m, D-5-H), 4.24 (1H, m, C-5-H), 4.21 (1H, m, E-5-H), 4.18 (1H, m, B-3-H), 4.18 (1H, m, F-3-H), 4.17 (1H, m, A-3-H), 4.17 (1H, m, E-3-H), 4.16 (1H, m, D-3-H), 4.14 (1H, m, A-5-H), 4.10 (1H, m, B-5-H), 3.98 (1H, m, D-2-H), 3.97 (1H, m, G-4-H), 3.96 (1H, m, G-5-H), 3.78 (1H, d-d, J=4.3, 10.4Hz, G-2-H), 1.34 (3H, d, J=7.0Hz, H₃ of D-5-CH₃), 1.15 (3H, d, J=6.7Hz, H₃ of E-5-CH₃), 1.12 (3H, d, J=6.7Hz, H₃ of A-5-CH₃), 1.11 (3H, d, J=6.7Hz, H₃ of C-5-CH₃), 1.08 (3H, d, J=6.7Hz, H₃ of B-5-CH₃), 1.06 (3H, d, J=6.4Hz, H₃ of F-5-CH₃), 1.04 (3H, d, J=6.7Hz, H₃ of G-5-CH₃)

### ¹³C-NMR(D₂ O)

δ 99.2 (G-1-C), 98.9 (C-1-C), 98.3 (B-1-C), 97.1 (E-1-C), 94.6 (F-1-C), 89.3 (A-1-C), 89.3 (D-1-C), 81.3 (F-4-C), 80.6 (B-4-C), 78.6 (A-4-C), 77.9 (G-3-C), 77.5 (C-4-C), 77.4 (E-4-C), 75.9 (B-3-C), 75.4 (A-2-C), 74.8 (F-2-C), 74.5 (B-2-C), 74.2 (D-3-C), 73.9 (A-3-C), 73.9 (D-2-C), 73.6 (C-2-C), 73.0 (D-4-C), 73.0 (E-2-C), 70.9 (C-3-C), 70.6 (D-5-C), 70.1 (G-4-C), 69.9 (E-3-C), 68.0 (B-5-C), 67.5 (A-5-C), 67.5 (E-5-C), 66.9 (C-5-C), 66.7 (G-5-C), 66.5 (F-3-C), 66.3 (F-5-C), 65.6 (G-2-C), 16.0 (C of C-5-CH₃), 15.9 (C of B-5-CH₃), 15.8 (C of E-5-CH₃), 15.8 (C of F-5-CH₃), 15.4 (C of G-5-CH₃), 15.3 (C of A-5-CH₃), 13.1 (C of D-5-CH₃)

Incidentally, the number of assignment of the peak of NMR is as shown in the following formula [II].

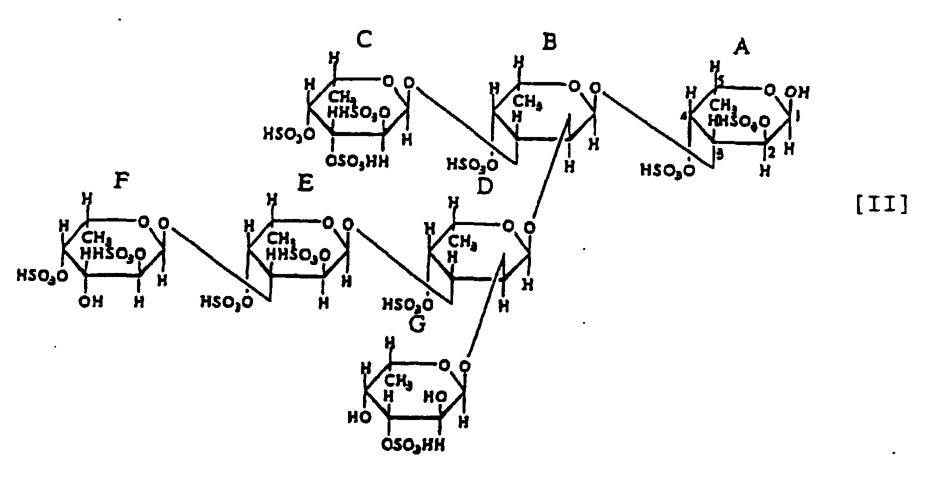

From the above result, it was found that this substance is the sulfated saccharide represented by the formula [III].

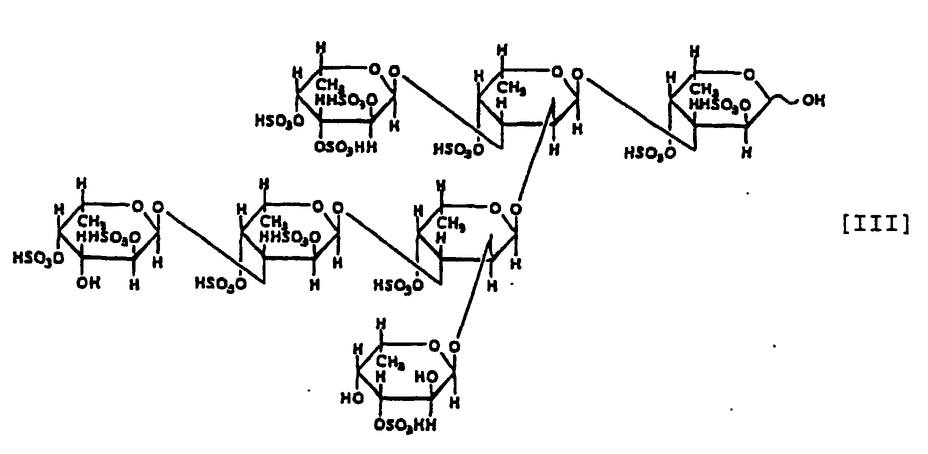

(3) The fraction no. 67 of DEAE-Cellulofine mentioned in Example 1-(2) was purified by entirely the same manner as in no. 63 to give a freeze-dried product.

As a result of an analysis by means of an HPLC, the resulting product was found to be a sulfated saccharide having higher molecular weight than no. 63 although, according to the analytical result by an NMR, the spectrum nearly the same as no. 63 was obtained.

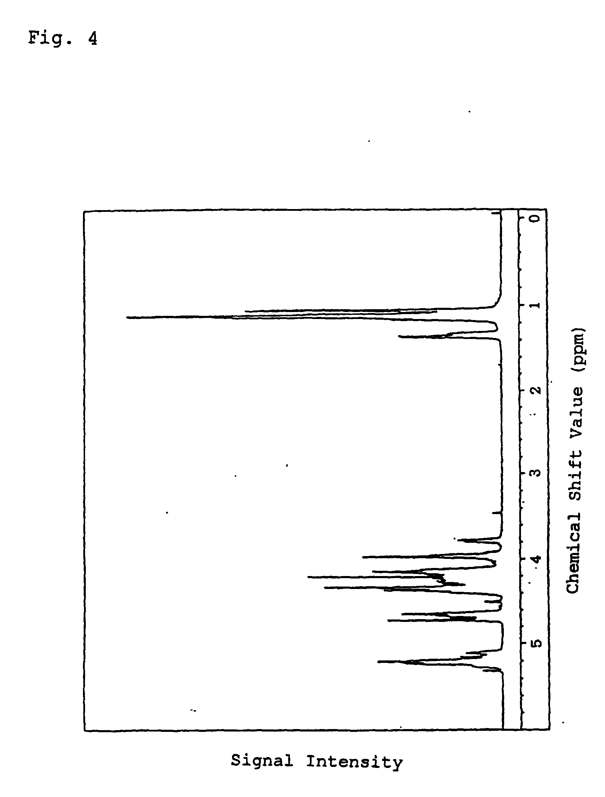

Fig. 4 shows the ¹H-NMR spectrum of the fraction no. 67 where heavy water was used as a solvent and the chemical shift value in ¹H-NMR was expressed in such a manner that the chemical shift value of dioxane was 3.53 ppm. Measurement was carried out at 60°C. Thus, Fig. 4 is a graph showing the ¹H-NMR spectrum of the fraction no. 67 where the ordinate indicates a signal intensity while the abscissa indicates a chemical shift value (ppm).

As a result, it was strongly suggested that the fraction no. 67 had a structure that several molecules of no.63 were bonded. Therefore, the fraction no. 67 was degraded by the endo-sulfated polysaccharide degrading enzyme mentioned in Example 1-(1) and the degraded product was analyzed by an HPLC whereupon many of the reaction product was eluted to the same position as the sulfated saccharide obtained from the fraction no. 63 of DEAE-Cellulofine mentioned in Example 1-(2).

Incidentally, the analytic condition for the HPLC was as follows.
Column: Shodex SB 802.5
Column temperature: 25°C
Solution: 50 mM sodium chloride containing 5 mM of sodium azide
Detection: differential refractometric detector (Shodex RI-71)

When molecular weights of the above-mentioned fraction no. 67 and no. 63 were measured by means of a gel filtration using pullulan (manufactured by Showa Denko) as a standard substance, no.63 had a molecular weight of about 8,500 based upon pullulan while no. 67 had a molecular weight of about 26,000 whereby it was found that the fraction no. 67 was a trimer of the sulfated saccharide of the fraction no. 63.

With regard to the bonding position of the repetition of the seven-sugar residue, the ¹H-NMR spectrum of the fraction no. 67 was checked in detail and it was found to be bonded at 3-position of fucose of F in the formula [II] by an α-(1→ 3) bond.

Similarly was prepared a pentamer of the sulfated saccharide represented by the formula [III], i.e. a sulfated saccharide (n = 5 in the formula [I]) from the degraded product of the sulfated polysaccharide of the present invention.

### Example 2.

(1) Dried *Kjellmaniella crassifolia* (2 kg) was powdered by a cutter mill (manufactured by Masuko Sangyo) equipped with a screen having a mesh of 1mm diameter, the resulting sea tangle chips were suspended in 20 liters of 80% ethanol, stirred at 25°C for three hours and filtered with a filter paper and the residue was well washed. The resulting residue was suspended in 40 liters of 20 mM sodium phosphate buffer (pH 6.5) containing 50 mM of sodium chloride which was heated at 95°C and the suspension was heated at 95°C for two hours with occasional stirring to extract the sulfated polysaccharide.

The suspended substance in the extract was filtered to give a filtrate while the residue after the filtration was washed with 3.5 liters of 100 mM sodium chloride to give an additional filtrate.

Both filtrates were combined, cooled down to 30°C, 3,000 units of alginic acid lyase (manufactured by Nagase Seikagaku Kogyo) were added, then 4 liters of ethanol were added and the mixture was stirred at 25°C for 24 hours. It was centrifuged, the resulting supernatant liquid was concentrated to 4 liters using an ultrafiltration system equipped with a holofiber having an excluding molecular weight of 100,000 and then an ultrafiltration was continued using 100 mM of sodium chloride containing 10% of ethanol until no more colored substance was filtered.

The precipitate formed in the non-filtrate was removed by centrifugation, the supernatant liquid was cooled down to 5°C and adjusted to pH 2.0 with 0.5N hydrochloric acid, the resulting precipitate consisting of protein, etc. was removed by centrifugation and the resulting supernatant liquid was promptly adjusted to pH 8.0 with 1N sodium hydroxide.

Then an ultrafiltration was carried out using an ultrafiltration system equipped with a holofiber having an excluding molecular weight of 100,000, the solvent was completely substituted with 20 mM sodium chloride, pH 8.0, then pH was again adjusted to 8.0, a centrifugation was carried out and a freeze-drying was conducted to prepare about 95 g of a sulfated polysaccharide.

(2) Ten grams of the dried sulfated polysaccharide mentioned in Example 2-(1) were dissolved in a buffer (pH 8.0) containing 100 mM of sodium chloride, 50 mM of calcium chloride and 50 mM of imidazole, 20 ml of the endo-sulfated polysaccharide degrading enzyme mentioned in Example 1-(1) were added thereto, the mixture was made to react at 25°C for 24 hours, the reaction solution was fully dialyzed using a dialyzing tube having an excluding molecular weight of 3,500, the dialyzed liquid (low-molecular substance) was concentrated and desalted by an electric dialyzer equipped with a membrane having an excluding molecular weight of 300 and a part of it was subjected to a gel filtration using Cellulofine GCL-90 (40 × 87 cm). The collected amount was 10 ml per tube.

The fraction near the no. 63 was analyzed by a cellulose acetate membrane electrophoresis and an HPLC and was found to show the same behavior as the sulfated saccharide having a structure [III) mentioned in Example 1-(2). Thus, the sulfated saccharide represented by the formula [III] was prepared from the sulfated polysaccharide. Similarly were prepared trimer and tetramer of the sulfated saccharide represented by the formula [III], i.e. the sulfated saccharides where n is 3 and 5, respectively, in the formula [I].

### Example 3.

A solution (10 liters) where the sulfated polysaccharide mentioned in Example 2-(1) was dissolved in a buffer (pH 8.0) containing 100 mM of sodium chloride, 50 mM of calcium chloride and 50 mM of imidazole to make the concentration of the sulfated polysaccharide 5 g per liter was prepared. To two liters of the said sulfated polysaccharide solution were added 30 ml of an endo-sulfated polysaccharide degrading enzyme mentioned in Example 1-(1), the mixture was treated with an ultrafiltration system equipped with a holofiber for ultrafiltration having an excluding molecular weight of 3,000 and an operation was conducted at 25°C to make the filtering speed 200 ml per hour. During the operation, the above-mentioned sulfated polysaccharide solution in the same amount as the filtered liquid amount was added to the enzymatic reaction solution. After the sulfated polysaccharide solution was added, only the above-mentioned buffer was added similarly.

After that, the same operation as in Example 1-(2) was conducted that the filtrate was concentrated and centrifuged, the resulting supernatant liquid was desalted, a precipitate was formed by calcium acetate and a supernatant fluid was obtained by centrifugation. The resulting supernatant liquid was purified by the same manner as in Example 1-(2) using DEAE-Cellulofine to prepare a sulfated saccharide represented by the formula [III].

### Example 4.

*Kjellmaniella crassifolia* (500 g) was finely cut, washed with 10 liters of 80% ethanol, stirred in 50 liters of 10% ethanol containing 1 mM of potassium chloride at 25°C for three days and filtered through a stainless wire net having a mesh of 32 µm to give an extract of the sulfated polysaccharide of the present invention.

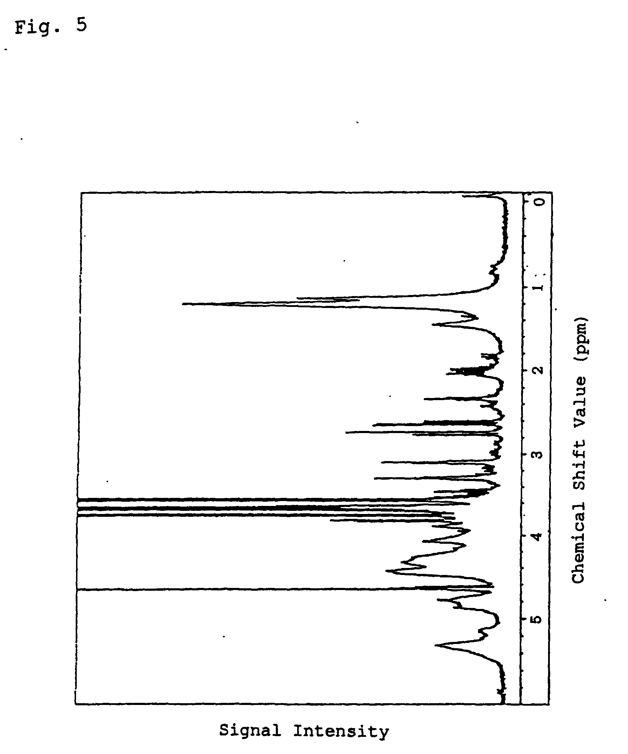

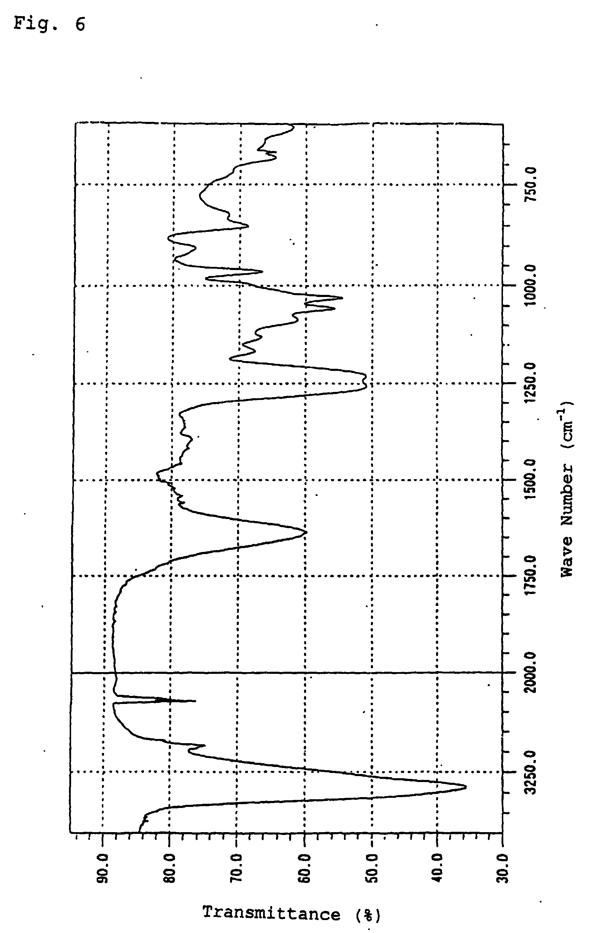

One hundred ml of the said extract were placed in a tube for the dialysis having an excluding molecular weight of 12,000 and dialyzed against 5 liters of water twice. The non-dialyzed fraction was freeze-dried to give the sulfated polysaccharide of the present invention and ¹H-NMR spectrum (Fig. 5) and infrared spectrum (IR) (Fig. 6) of the said sulfated polysaccharide were measured. Thus, Fig. 5 is a graph showing the ¹H-NMR spectrum of the sulfated polysaccharide of the present invention where the ordinate indicates a signal intensity while the abscissa indicates a chemical shift values (ppm). Incidentally, heavy water was used as a solvent and, with regard to the chemical shift value in the ¹H-NMR, the chemical shift value of HOD was expressed at 4.65 ppm. Fig. 6 is a graph showing an IR spectrum of the sulfated polysaccharide of the present invention by an KBr method where the ordinate indicates a transmittance (%) while the abscissa indicates a wave number (cm⁻¹). Incidentally, the IR spectrum was measured by an FTIR-8000 PC Infrared Spectrophotomer (manufactured by Shimadzu).

As shown in Fig. 5, the ¹H-NMR of the sulfated polysaccharide of the present invention is almost same as that of the sulfated saccharide shown in Fig. 4. Thus, the sulfated polysaccharide of the present invention is a sulfated polysaccharide where the sulfated saccharide represented by the formula [I] as an essential component of the constituting sugar.

A 4M sodium chloride was added to the extract of the sulfated polysaccharide of the present invention, the mixture was mixed under vigorous stirring, the final concentration was made a 1M sodium chloride solution, and this was analyzed by an HPLC to measure the molecular weight of the sulfated polysaccharide of the present invention. The sulfated polysaccharide of the present invention showed an average molecular weight of about 13,000,000 based upon the pullulan standard substance. Condition for the HPLC was as follows.
Apparatus: HPLC of type L-6200 (manufactured by Hitachi)
Column: Shodex SB-806HQ (8 × 300 mm) (manufactured by Showa Denko)
Eluent: 50 mM sodium chloride
Detection: Differential refractometric detector, Shodex RI-71 (manufactured by Showa Denko)
Column temperature: 25°C

When the sulfated polysaccharide solution of the present invention was treated with an endo-sulfated polysaccharide degrading enzyme mentioned in Example 1-(1), a sulfated saccharide represented by the formula [I] was detected.

### Example 5.

*Kjellmaniella crassifolia* (500 g) was finely cut, washed with 10 liters of 80% ethanol, stirred in 50 liters of 10% ethanol containing 1 mM of potassium chloride at 25°C for two days in a container having an inner diameter of 40 cm at the rate of 200 rpm to extract the sulfated-fucose-containing sulfated polysaccharide cross-linked with protein of marine algae according to the present invention. The extract showed a strong viscoelasticity and showed a Weissenberg effect where the extract was wound up along the stirring axis.

Usually, the content of the sulfated-fucose-containing sulfated polysaccharide in *Kjellmaniella crassifolia* was around 5% of the dried marine algae at maximum and, therefore, the content of the sulfated-fucose-containing sulfated polysaccharide in the present extract is 0.05% at maximum.

However, a commercially available aqueous solution of the sulfated-fucose-containing sulfated polysaccharide (Fucoidan; manufactured by Sigma)does not show viscoelasticity at all not only at 0.05% but even at the concentration of as high as 2% and has an entirely different property from the sulfated-fucose-containing sulfated polysaccharide which is cross-linked with protein of algae according to the present invention.

When the protein content in the present extract was measured by a Protein Assay (manufactured by Bio-Rad), it was found to be 7.5 µg/ml.

When the present extract was treated with Actinase E (a kind of protease; manufactured by Kaken Pharmaceutical) in a concentration of 0.1 mg/ml, there was a significant decrease in its viscoelasticity.

Moreover, when 5 ml of the present extract was treated with 10 µl of a solution containing an endo-sulfated-fucose-containing polysaccharide degrading enzyme produced by Alteromonas sp. SN-1009 (FERM BP-5747), the viscoelasticity completely disappeared.

From the above result, it has been found that the substance which is responsible for the viscoelasticity of an extract of brown algae is a sulfated-fucose-containing sulfated polysaccharide cross-linked with protein and that, when the sulfated-fucose-containing sulfated polysaccharide of brown algae is extracted under a specific condition, the sulfated-fucose-containing sulfated polysaccharide cross-linked with protein according to the present invention can be efficiently extracted.

When the resulting extract was applied to skin, a strong sliminess was firstly noted but, when gently rubbed thereinto, the sliminess was adsorbed to the skin giving no stickiness and the skin was moisturized whereby the extract was found to be quite useful as a cosmetic material for skin care.

### Example 6.

(1) *Kjellmaniella crassifolia* (50 g) was finely cut, washed with 2 liters of 80% ethanol and stirred with 5 liters of aqueous solution containing 1 mM of potassium chloride and 0.075% of ethyl paraben in a container having an inner diameter of 20 cm at 25°C for two days at the rate of 200 rpm to extract the sulfated-fucose-containing sulfated polysaccharide cross-linked with protein according to the present invention.

The extract had a strong viscoelasticity and showed a Weissenberg effect where the extract was wound up along the stirring axis. The property of this extract was the same as that of the extract of Example 5. This extract is effective as cosmetics for the people whose skin is sensitive to ethanol.

(2) Dried *Kjellmaniella crassifolia* (2 kg) was powdered by a cutter mill (manufactured by Masuko Sangyo) equipped with a screen having a mesh of 1mm diameter, the resulting sea tangle chips were suspended in 20 liters of 80% ethanol, the suspension was stirred at 25°C for three hours and filtered and the residue was well washed. The resulting residue was suspended in 40 liters of a 20 mM sodium phosphate buffer (pH 6.5) containing 50 mM of sodium chloride which was heated at 95°C and the suspension was heated at 95°C for two hours with occasional stirring to extract a sulfated-fucose-containing sulfated polysaccharide.

The suspended matter in the extract was filtered to prepare a filtrate while the residue after filtration was washed with 3.5 liters of a 100mM sodium chloride to give an additional filtrate. The extract prepared as such was cooled down to 25°C. This extract contained more sulfated-fucose-containing sulfated polysaccharide than the extracts mentioned in Example 5 and in Example 6-(1) but did not show a viscoelasticity. Moreover, this extract did not show a strong viscoelasticity even after a full desalting by an ultrafiltration system equipped with a holofiber having an excluding molecular weight of 100,000 although it showed a viscosity specific to the sulfated polysaccharide. Thus, it has been found that, when the extracting temperature is too high, the sulfated-fucose-containing sulfated polysaccharide cross-linked with protein according to the present invention cannot be prepared.

(3) *Kjellmaniella crassifolia* (500 g) was finely cut, washed with 10 liters of 80% ethanol and stirred in 50 liters of 10% ethanol containing 1 mM potassium chloride in a container having an inner diameter of 40 cm at 25°C for two days at the rate of 800 rpm to extract the sulfated-fucose-containing sulfated polysaccharide. Viscoelasticity of the extract was weak and, even during stirring, no Weissenberg effect was noted. Although the extract showed a moisturizing effect specific to sulfated-fucose-containing sulfated polysaccharide when applied to skin, it rarely had sliminess specific to the sulfated-fucose-containing sulfated polysaccharide cross-linked with protein according to the present invention and the feel on actual use was entirely different. However, concentration of the protein contained therein was 7.5 µg/ml and was same as that in the extract of Example 5. Therefore, it has been found that, when the stirring speed is too high or, in other words, when a shearing force is strongly applied upon stirring, the cross-link between protein and sulfated-fucose-containing sulfated polysaccharide is destroyed.

(4) When the extract obtained in Example 6-(1) was stirred in a container having an inner diameter of 20 cm at the stirring rate of 600 rpm at room temperature for 18 hours, the relative viscoelasticity decreased from 2.0 to 1.2. When a 1% aqueous solution of gelatin in various amounts was added to the sulfated-fucose-containing sulfated polysaccharide having a reduced viscoelasticity, there was a recovery in the viscoelasticity. The relation between the viscoelasticity and the final concentration of gelatin will be given in the following Table 1.

In the following Table 1, the viscoelasticity is shown in relative values. Thus, when the test solution was vertically flown down by gravity from a silicone tube having an inner diameter of 2 mm and the test solution fallen down at about 1-5 cm downward from the outlet was pushed in a horizontal direction by a glass rod, the maximum distance (cm) which was able to be pushed without cutting the flow was defined as the relative viscoelasticity. Incidentally, the distance from the liquid surface of the test solution to the outlet of the test solution was 20-21 cm and the relative viscoelasticity of water was 0.

**Table 1**

| Final Concentration of Gelatin (%) | Relative Viscoelasticity |
|---|---|
| 0 | 1.2 |
| 0.01 | 1.8 |
| 0.012 | 1.8 |
| 0.02 | 3.5 |
| 0.03 | 1.5 |
| 0.05 | 0 |

From the above result, it has been found that the viscoelasticity of the sulfated-fucose-containing sulfated polysaccharide cross-linked with protein of marine algae according to the present invention lowers by stirring with a strong shearing force but that, when an appropriate amount of gelatin is added at an appropriate pH or under the condition of an appropriate salt concentration, the viscoelasticity can be recovered whereby a viscoelastic product can be obtained. Incidentally, the viscoelastic product has a sol-like property showing a transparent jelly-like appearance.

When the adding amount of gelatin was too much, the viscoelasticity disappeared and, therefore, it has been found that the amount is to be increased or decreased depending upon the amount of the sulfated-fucose-containing sulfated polysaccharide.

### Example 7.

When an extract of Example 5 was applied to mouth of a lady of 80 years old where secretion of saliva was so insufficient that mouth was significantly dry and was dificult to be opened and shut and any preparation had shown no improvement in the symptom, there was an improvement in drying of mouth, chapping of lips, etc. and difficulties in opening and shutting of the mouth was significantly improved. Moreover, the effect lasted even when the extract was applied for continued period of three months or longer.

### Example 8.

(1) *Kjellmaniella crassifolia* (500 g) was finely cut, washed with 10 liters of 80% ethanol and stirred in 50 liters of 10% ethanol containing 1 mM potassium chloride in a container having an inner diameter of 40 cm at 25°C for two days at the rate of 120 rpm to extract the sulfated-fucose-containing sulfated polysaccharide. The extract had a strong viscoelasticity and showed a Weissenberg effect where the extract was wound up along the stirring axis. The extract was filtered through a stainless wire net having a mesh of 32 µm to prepare a solution of the sulfated-fucose-containing sulfated polysaccharide having a high viscoelasticity.

To 46 liters of the said solution containing the sulfated-fucose-containing sulfated polysaccharide was added, with stirring, one liter of a palm oil solution where 1 g of palm oil (manufactured by Kao; for cosmetic use) was dissolved in one liter of ethanol and then one liter of glycerol was added thereto to prepare a cosmetic lotion. The resulting cosmetic lotion showed both moisturizing effect due to the highly viscoelastic sulfated-fucose-containing sulfated polysaccharide and anti-drying effect due to the palm oil wherein the palm oil was dispersed uniformly and efficiently without adding detergent, whereby the said cosmetic lotion showed a good feel on use without stickiness of the oil and with a good spread.
Another cosmetic lotion was manufactured similarly using coconut oil (manufactured by Kao; for cosmetic use) instead of palm oil and the resulting cosmetic lotion was found to have a good feel on use as well.

(2) To the extract prepared in Example 6-(3) were added gelatin and perfume to make the final concentrations 0.02% whereupon a cosmetic lotion containing gelatin was obtained. A cosmetic lotion containing collagen was obtained in a similar manner by addition of collagen. Each of the cosmetic solutions contains a highly viscoelastic sulfated-fucose-containing sulfated polysaccharide and, as a result of synergism with the protein added thereto, it was a cosmetic lotion having an excellent moisturizing property and a good spread.

As such, when the above-mentioned cosmetic lotion was used, the feel on use was good with a smooth touch due to its viscoelasticity and, moreover, when an appropriate amount was applied to skin, it showed a property that sliminess was immediately adsorbed to the skin. Furthermore, there was no stickiness after use and that was a very favorable property as cosmetics.

### Example 9.

The extract of the sulfated polysaccharide of the present invention prepared in Example 4 was used as a cosmetic lotion. This cosmetic lotion had a good feel on use due to smooth touch and, when applied to skin, the sliminess was immediately adsorbed with the skin. Moreover, there was no stickiness after use and that was a very favorable property as cosmetics. Furthermore, when this cosmetic lotion was used, there was an effect that spots on the face and back of the hand become light in color.

### Advantage of the Invention

In accordance with the present invention, sulfated saccharide, sulfated polysaccharide or a salt thereof useful as pharmaceuticals or reagents for the study of sugar chain engineering is offered. The said sulfated saccharide, sulfated polysaccharide or salt thereof is quite useful as a material for cosmetics due to its water-holding ability, etc.

The present invention also offers a sulfated polysaccharide having a high viscoelasticity and a method for manufacturing the same. Moreover, a pharmaceutical agent and cosmetics containing the said highly viscoelastic sulfated polysaccharide is offered as well. The pharmaceutical agent of the present invention is useful as an intraoral agent for example. Furthermore, the highly viscoelastic sulfated polysaccharide of the present invention has not only excellent water-holding ability and lubricity but also a high affinity to keratinous layer of skin due to its hydrophobicity and a very good compatibility with skin whereby the sulfated polysaccharide, particularly the highly viscoelastic sulfated-fucose-containing polysaccharide, of the present invention is quite useful as a material for cosmetics.

## Claims

1. A sulfated saccharide represented by the following formula [I] or a sulfated polysaccharide where the said sulfated saccharide is an essential component of the constituting sugar as well as a salt thereof. 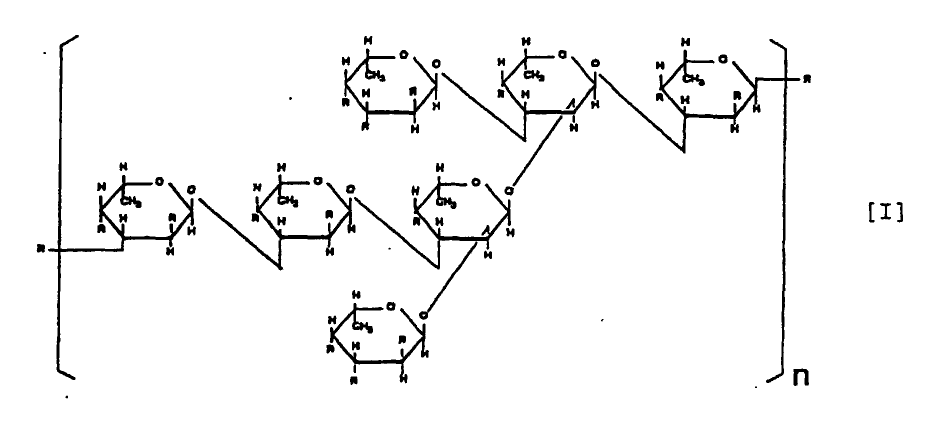 (In the formula, R is OH or OSO₃H; and n is an integer of 1-5.)

2. A sulfated polysaccharide according to claim 1 which the sulfated polysaccharide can be obtained from Marine algae belonging to Order of *Laminariales.*

3. A sulfated polysaccharide according to claim 1 or 2 which the sulfated polysaccharide is cross-linked with a polycationic substance.

4. A sulfated polysaccharide according to claim 3 which the polycationic substance is a protein.

5. A sulfated polysaccharide according to claim 4 which the protein is a protein derived from marine algae and/or a protein other than derived from marine algae.

6. A sulfated polysaccharide according to claim 5 which the protein is collagen and/or gelatin.

7. A method for the manufacture of a sulfated polysaccharide cross-linked with a polycationic substance which is characterized in including an extracting step under a non-destructive condition for a cross-link between polycationic substance and sulfated polysaccharide from algae.

8. A method for the manufacture of a sulfated polysaccharide cross-linked with a polycationic substance which is characterized in adding a polycationic substance having higher isoelectric point than the pH of a solution of sulfated polysaccharide to the said solution.

9. A method of heightening a viscoelasticity of a sulfated polysaccharide, characterized in that, a polycationic substance is added to a sulfated polysaccharide.

10. A composition which contains at least a sulfated polysaccharide having a low viscoelasticity and a polycationic substance.

11. A pharmaceutical agent, characterized in that, a sulfated polysaccharide which is cross-linked with a polycationic substance is contained as an effective component.

12. A pharmaceutical agent according to claim 11 which the pharmaceutical agent is an intraoral preparation.

13. A lubricant, characterized in that, a sulfated polysaccharide which is cross-linked with a polycationic substance is contained as an effective component.

14. Cosmetics, characterized in that, a sulfated polysaccharide which is cross-linked with a polycationic substance is contained as an effective component.
